(19)

**Europäisches Patentamt**
**European Patent Office**
**Office européen des brevets**

(11) **EP 1 973 578 B1**

(12) **EUROPEAN PATENT SPECIFICATION**

(45) Date of publication and mention
of the grant of the patent:
**03.04.2013 Bulletin 2013/14**

(21) Application number: **06788007.0**

(22) Date of filing: **20.07.2006**

(51) Int Cl.:
*A61L 2/10* *(2006.01)*    *A61L 9/20* *(2006.01)*

(86) International application number:
**PCT/US2006/028227**

(87) International publication number:
**WO 2007/081401 (19.07.2007 Gazette 2007/29)**

(54) **USE OF ULTRAVIOLET GERMICIDAL IRRADIATION IN HEALTH CARE ENVIRONMENTS**

VERWENDUNG VON ULTRAVIOLETTER GERMIZIDER STRAHLUNG IN DER GESUNDHEITSFÜRSORGE

UTILISATION DE L'IRRADIATION ULTRAVIOLETTE GERMICIDE DANS LES ENVIRONNEMENTS DE SANTÉ

(84) Designated Contracting States:
**AT BE BG CH CY CZ DE DK EE ES FI FR GB GR HU IE IS IT LI LT LU LV MC NL PL PT RO SE SI SK TR**

(30) Priority: **14.01.2006 US 758638 P**

(43) Date of publication of application:
**01.10.2008 Bulletin 2008/40**

(73) Proprietor: **Optimus Licensing AG**
**6300 Zug (CH)**

(72) Inventor: **MANGIARDI, John R.**
**Greenwich, CT 06830 (US)**

(74) Representative: **Manitz, Finsterwald & Partner GbR**
**Martin-Greif-Strasse 1**
**80336 München (DE)**

(56) References cited:
**WO-A2-03/101498**    **US-A1- 2004 120 845**
**US-A1- 2005 035 301**    **US-A1- 2005 123 436**
**US-A1- 2005 207 951**    **US-B1- 6 296 775**
**US-B1- 6 893 610**    **US-B2- 6 911 177**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND OF THE INVENTION -- FIELD OF INVENTION

The present invention relates to methods for producing sanitary health care environments, such as the operating room or other ambulatory treatment facilities, by utilizing ultraviolet irradiation and ozone to destroy viruses, bacteria, and fungus.

BACKGROUND OF THE INVENTION

Hospital-acquired infections are responsible for tens of thousands of fatalities every year. These nosocomial infections are especially difficult to treat since increasingly, the strains are drug resistant. For example, it is estimated that 50% of staphylococcal strains (which can cause infection in post-op incisions) are resistant to all antibiotics currently in use. One approach to the control of such infectious agents involves the use of ultraviolet radiation in the "C" band range of around 200-280 nm. This technique is known as ultraviolet germicidal irradiation (UVGI). It is not a new technique, having been used as early as 1909 to disinfect the municipal water supply of Marseilles, France. More recently, it has been used to control contamination of air handlers and in isolation-rooms, especially for tuberculosis patients in hospitals. For example, US 6,911,177 B2 discloses a method of sterilizing the air in a room comprising the steps of evacuating personnel from said room; emitting ultraviolet radiation in the UV-C band range from a room sterilizer; and discontinuing emission of ultraviolet radiation when the air in said room is sterile for medical use; wherein said ultraviolet radiation is generated by an ultraviolet radiation source. US 2005/0123436 A1 teaches a method of decontamination of a surgical suite in a hospital using an ozone generator. The space is scanned to ensure that no personnel are present in the room. A lock down procedure is instituted to present anyone from entering the space during the treatment. Ozone destruction unit can be employed to seduce ozone level to acceptable level. Other sterilization techniques include using ozone, but the ability to use ozone is limited because exposure to ozone is unhealthful for humans. Further, many decontamination means are incapable of providing a truly sterile environment. For example, while water-purifiers exist for water coming into a hospital sink, there is no effective method for sterilizing sink-traps. Sink-traps are a major source of drug-resistant, dangerous biologies. Hospitals are undertaking extensive infection-control programs. The operating room should be an active part of such efforts; therefore, a method and/or apparatus that provides a sterile hospital room environment would be of benefit.

UVGI can be utilized in the operating room environment to help sterilize contaminations from a variety of sources including infected equipment brought in, the surgical patient, the surgical team, and outside air.

As contaminants are brought into contact with UVC-band radiation, both the membrane and nucleus are penetrated. The UVC-band light then breaks up the molecular bonds of the DNA of the microorganism, thereby killing the microbe or inhibiting its reproduction. Spores and some bacteria tend to be somewhat more resistant than viruses, but all succumb to some dose of radiation after a period of time. Further, organic compounds which are exposed to UVC-band radiation are placed in an excited-energy state. In combination with a reactive molecule such as ozone, the excited organic compound or organism is more likely to react, leading to the destruction of the compound/organism, usually through an oxidative pathway. Ozone has been shown to be an effective sterilizer but is a strong irritant and unhealthy for humans. As such, an invention that can effectively combine ozone and UV sterilization while minimizing exposure to humans would provide a means for a sterile environment.

The survival probability of bacteria after being exposed to UVGI depends both on the irradiance as well as the exposure time in the general form of the following formula:

$$\% Survival = 100 \times e^{-kIt} \tag{1}$$

where in formula (1):

e = Napier's constant, approximately equal to 2.7183 and defined such that the natural logarithm of e is one.
I = UV irradiance in microwatts per square centimeter:

$$\frac{\mu W}{cm^2}$$

t = time of UV exposure in seconds

k = microbe susceptibility factor in square centimeters per microwatt seconds:

$$\frac{cm^2}{\mu W \times s}$$

It is the "k" factor that differentiates the irradiation time necessary to kill a particular microorganism. The prior art utilizes UV irradiation to provide a lethal dose of radiation and requires hospital personnel to leave the environment in which sterilization is occurring. Since a single organism could require a lengthy dose of radiation, because of a low k factor, proper use of such devices may involve the room being vacant, and hence unusable, for a long period of time.

Current devices, such as the air-handling system of Welch in U.S. Pat. No. 5,086,692 or the UV room sterilizer of Deal in U.S. Pat. No. 6,911,177, provide more sanitary or sterile areas of the hospital room, but cannot reach all spaces even with reflected UV rays. For example, cabinetry surfaces that are not in the line of sight of a directed UV emission or reflected UV emission will not sterilize. With the necessity that hospital operating room environments be as sterile as possible, a device that can provide at least 99.99% sterility on all exposed surfaces in a hospital room would be of benefit. A room is 99.99% sterile when 99.99% of all (previous to sterilization) known pathogens are destroyed. A pathogen is any fungus, virus, or bacterium typically found in hospital room environments.

It is an object of this invention to provide a method according to the claims 1-4.

SUMMARY OF THE INVENTION

The operating room or other health care environment of this invention uses UVGI to control contamination in a hospital room environment. Sanitization is provided by a ceiling-mounted UV/ozone sterilizer. This device, optionally used in combination with other traditional devices such as an air-duct sanitizer or floor sanitizer, provide means for an improved method of room sanitation. The device provides full-room sanitation for when operating room personnel are not present when used in combination with an airtight operating room.

The ceiling device utilizes UV tubes to provide an irradiation source and an ozone generator to provide ozone. More than one tube is provided on each device and the tubes are segregated into an up region and a down region. The up region irradiates ceiling and wall surfaces in its light of sight and surfaces reached by reflection. The down region is designed to irradiate all other surfaces within its line of sight and those that are reached by reflection of UV rays. When used, the room is evacuated of personnel. Ozone is generated during the emission of UV light. The UV light and ozone work alone and synergistically to destroy pathogens. Particularly, the ozone is able to reach non-reflected surfaces because of its gaseous nature. placed near the sink trap transparent portion. The above device may be used in conjunction with other known sterilization means, such as an air duct sanitizer or a water-source sanitizer to provide an improved method of room sterilization.

BRIEF DESCRIPTION OF THE DRAWINGS

The present invention can best be understood in connection with the accompanying drawings. It is noted that the invention is not limited to the precise embodiments shown in drawings, in which:

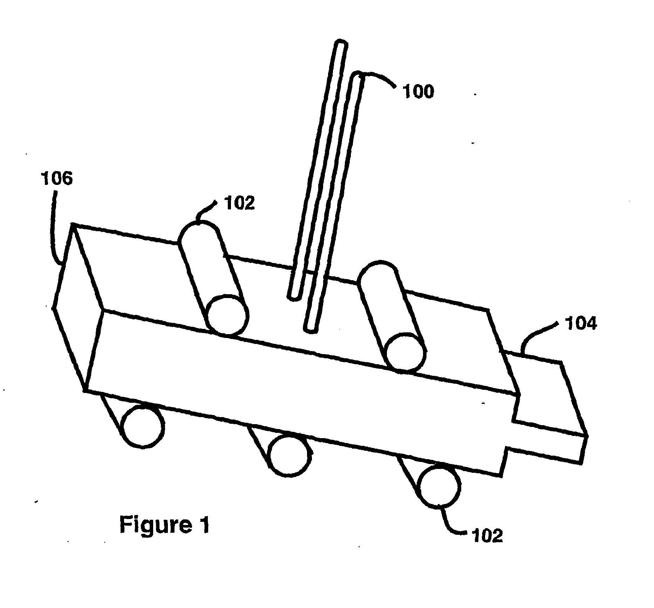

• Fig. 1 is a perspective view of one possible embodiment of the UV/ozone, ceiling-mounted sterilizer.

BRIEF DESCRIPTION OF REFERENCE NUMERALS

100 ceiling mounts; 102 UV tube; 104 ozone generator; 106 room sterilizer;

DETAILED DESCRIPTION OF THE INVENTION

Fig. 1 is a perspective view of one possible embodiment of the UV/ozone, ceiling-mounted sterilizer. When the device is mounted within an operating room as by mounts **100,** preferably on a ceiling, and the airtight room evacuated of personnel, it is powered on. By providing emission of UV rays from the UV bulbs **102** in conjunction with ozone from the ozone generator **104,** the room sterilizer **106** sterilizes the room. The method is superior to UV only sterilization techniques because the production of ozone sterilizes surfaces not in the line of sight of direct or reflected UV rays. Further, the method is used in conjunction with an airtight room, thereby allowing high concentrations of ozone to be generated. After a sufficient period of time has elapsed, the generator may be turned of, such as by an outside the room power switch. Ozone decomposes naturally into harmless components; therefore, after a suitable waiting period, the room is sterilized to at least 99.99% sterility and personnel may reenter.

**Claims**

1. A method of sterilizing the surfaces of an air-tight hospital room comprising the steps of:

   (i) evacuating personnel from said room;
   (ii) emitting ozone gas in conjunction with continuously emitting ultraviolet radiation from a ceiling-mounted room sterilizer (106); and
   (iii) discontinuing emission of ozone gas and ultraviolet radiation when the surfaces of said room are sterile for medical use;

   wherein

   said ultraviolet radiation is generated by an ultraviolet radiation source (102) in said ceiling-mounted room sterilizer (106); said ultraviolet radiation is in the UV-C band range; and
   said ozone gas is generated by an independent ozone generator (104) in said ceiling-mounted room sterilizer (106).

2. The method of claim 1 in which said ceiling-mounted room sterilizer (106) has a detector adapted to sense the presence of persons within the room and further adapted to prevent functioning of said ceiling-mounted room sterilizer (106) when said persons are within the room.

3. The method of claim 1 or 2 in which said ceiling-mounted room sterilizer (106) has an alarm that sounds when personnel are in the room and the room sanitizer is on or there are levels of ambient ozone dangerous to humans.

4. The method of claim any proceeding claim in which said room is sterilized until 99,99% of pathogens are destroyed.

**Patentansprüche**

1. Verfahren zum Sterilisieren der Oberflächen eines luftdichten Krankenhausraums, mit den Schritten:

   (i) Räumen von Personal aus dem Raum;
   (ii) Emittieren von Ozongas in Verbindung mit kontinuierlichem Emittieren ultravioletter Strahlung von einer an der Decke montierten Raumsterilisationseinrichtung (106); und
   (iii) Unterbrechen der Emission von Ozongas und ultravioletter Strahlung, wenn die Oberflächen des Raums zum medizinischen Gebrauch steril sind;

   wobei
   die ultraviolette Strahlung durch eine Quelle (102) für ultraviolette Strahlung in der an der Decke montierten Raumsterilisationseinrichtung (106) erzeugt wird; die ultraviolette Strahlung in dem Gebiet des UV-C-Bandes ist; und das Ozongas durch einen unabhängigen Ozongenerator (104) in der an der Decke montierten Raumsterilisationseinrichtung (106) erzeugt wird.

2. Verfahren nach Anspruch 1,
   wobei die an der Decke montierte Raumsterilisationseinrichtung (106) einen Detektor aufweist, der derart angepasst ist, die Anwesenheit von Personen in dem Raum zu erfassen, und ferner derart angepasst ist, eine Funktion der an der Decke montierten Raumsterilisationseinrichtung (106) zu verhindern, wenn sich die Personen in dem Raum befinden.

3. Verfahren nach einem der Ansprüche 1 oder 2,
   wobei die an der Decke montierte Raumsterilisationseinrichtung (106) einen Alarm aufweist, der ertönt, wenn sich Personal in dem Raum befindet und die Raumdesinfektionseinrichtung eingeschaltet ist oder Niveaus von Umgebungsozon, die für Menschen gefährlich sind, vorhanden sind.

**4.** Verfahren nach einem der vorhergehenden Ansprüche,
wobei der Raum sterilisiert wird, bis 99,99 % von Pathogenen zerstört sind.

**Revendications**

**1.** Procédé pour stériliser les surfaces d'une chambre d'hôpital étanche à l'air, comprenant les étapes consistant à :

(i) évacuer le personnel hors de ladite chambre ;
(ii) émettre de l'ozone gazeux en combinaison avec une émission en continu de rayonnement ultraviolet depuis un stérilisateur de chambre monté au plafond (106) ; et
(iii) arrêter l'émission d'ozone gazeux et l'émission de rayonnement ultraviolet quand les surfaces de ladite chambre sont stériles en vue d'une utilisation médicale ;

dans lequel
ledit rayonnement ultraviolet est généré par une source de rayonnement ultraviolet (102) dans ledit stérilisateur de chambre monté au plafond (106) ; ledit rayonnement ultraviolet est dans la bande UV-C ; et ledit ozone gazeux est généré par un générateur d'ozone indépendant (104) dans ledit stérilisateur de chambre monté au plafond (106).

**2.** Procédé selon la revendication 1, dans lequel ledit stérilisateur de chambre monté au plafond (106) possède un détecteur adapté à détecter la présence de personnes dans la chambre, et en outre adapté à empêcher le fonctionnement dudit stérilisateur de chambre monté au plafond (106) quand lesdites personnes se trouvent dans la chambre.

**3.** Procédé selon la revendication 1 ou 2, dans lequel ledit stérilisateur de chambre monté au plafond (106) possède une alarme qui retentit quand du personnel est dans la chambre et que le stérilisateur de chambre est en marche ou qu'il se présente des niveaux d'ozone ambiant dangereux aux êtres humains.

**4.** Procédé selon l'une quelconque des revendications précédentes, dans lequel ladite chambre est stérilisée jusqu'à destruction de 99,99 % d'agents pathogènes.

Figure 1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6911177 B2
- US 20050123436 A1
- US 5086692 A, Welch
- US 6911177 B, Deal